# EUROPÄISCHE PATENTSCHRIFT

(11) **EP 2 180 885 B1**
(45) Veröffentlichungstag und Bekanntmachung des Hinweises auf die Patenterteilung: **29.01.2014**
(21) Anmeldenummer: 08841666.4
(22) Anmeldetag: 21.08.2008
(51) Int. Cl.: A61K 9/52, A61K 9/20, A61K 36/064

(54) **ARZNEIMITTEL MIT HEFE**
PHARMACEUTICAL PRODUCT COMPRISING YEAST
MÉDICAMENT COMPRENANT DE LA LEVURE

(30) Priorität: 01.09.2007 DE 102007041588
(43) Veröffentlichungstag der Anmeldung: 05.05.2010
(73) Patentinhaber: LTS LOHMANN Therapie-Systeme AG, 56626 Andernach (DE)
(72) Erfinder: ASMUSSEN, Bodo, 56170 Bendorf (DE); SCHILLER, Christiane, 18435 Stralsund (DE); WEITSCHIES, Werner, 17498 Neuenkirchen (DE); GARBACZ, Gregor, 17489 Greifswald (DE); OMER-ADAM, Mahmoud A., 38102 Braunschweig (DE); NAGEL, Stefan, 17489 Greifswald (DE)
(74) Vertreter: Michalski Hüttermann & Partner Patentanwälte
(86) Internationale Anmeldenummer: PCT/EP2008/006897
(87) Internationale Veröffentlichungsnummer: WO 2009/052888

(56) Entgegenhaltungen:
- WO-A-02/100391
- WO-A-02/102415
- WO-A-2006/024638
- US-A1- 2006 003 003

## Beschreibung

Die vorliegende Erfindung betrifft ein Arzneimittel mit steuerbarer Abgabe des in ihr enthaltenen Arzneistoffs, das zur Gärung befähigte Hefe enthält, deren Kohlendioxidproduktion den Arzneistoff aus dem Arzneimittel freisetzt.

Bei der medikamentösen Therapie zahlreicher Erkrankungen, beispielsweise bei Infektionskrankheiten, Störungen des Herz- und Kreislaufsystems, Allergien, Schmerzzuständen oder Störungen im Hormonhaushalt, ist die Aufrechterhaltung eines konstanten Arzneistoffspiegels im Blut oder Gewebe für einen längeren Zeitraum erwünscht. Um dies zu erreichen, werden insbesondere für Arzneistoffe, die keine lange Halbwertszeit im Plasma haben, Arzneiformen mit modifizierter Wirkstofffreisetzung eingesetzt. Hierzu zählen Systeme mit verlängerter Wirkstofffreisetzung, Systeme mit verzögerter Freisetzung und Systeme mit pulsatiler Wirkstoffabgabe. Ein Hauptziel ist dabei die Reduktion der intra- und interindividuellen Variabilität der Plasmaspiegel. Insbesondere sollen Plasmaspiegelspitzen und damit ein höheres Nebenwirkungsrisiko vermieden werden. Darüber hinaus können mit diesen Arzneiformen Fehler bei der Einnahme vermieden und die Compliance der Patienten verbessert werden, insbesondere durch die einfache Handhabung und die reduzierte Einnahmefrequenz dieser Arzneiformen.

Auch bei der medikamentösen Behandlung von Krankheitszuständen, die circadianen Rhythmen unterliegen und bei denen die Krankheitssymptome bevorzugt zu bestimmten Tageszeiten gehäuft oder verstärkt auftreten, werden Arzneiformen mit modifizierter Wirkstofffreisetzung eingesetzt.

Aus dem Stand der Technik sind vielfältige Möglichkeiten bekannt, mit denen die Arzneistoffabgabe aus einem Arzneimittel beeinflußt werden kann. Beispielsweise kann der Arzneistoff in seinen physikalisch-chemischen Eigenschaften so modifiziert werden, daß seine Lösungsgeschwindigkeit verändert wird. Hierzu zählen u. a. die Bindung des Arzneistoffs an ein Ionenaustauscherharz oder die Wahl der Teilchengröße. Pharmazeutisch-technologische Maßnahmen betreffen die Wahl der Hilfsstoffe und die Ausgestaltung der Arzneiform. So läßt sich eine Modifikation der Arzneistofffreisetzung durch die Eigenschaften von Überzügen, Matrixbildnern oder den Aufbau der Arzneiform erreichen. Generell können im Hinblick auf die Kinetik der Wirkstofffreisetzung diffusionsgesteuerte, erosionsgesteuerte und osmotisch gesteuerte Abgabesysteme unterschieden werden.

Die Freisetzung des Arzneistoffs erfolgt bei den bekannten Arzneimitteln für eine gesteuerte Arzneistofffreisetzung jedoch nicht unabhängig von den äußeren Bedingungen, so daß die Arzneistofffreisetzung unvorhersehbaren Schwankungen unterliegen kann. Die der vorliegenden Erfindung zugrundeliegende Aufgabe bestand daher darin, ein Arzneimittel zu entwickeln, bei dem die Arzneistofffreisetzung möglichst unabhängig von den äußeren Bedingungen erfolgt.

Die Aufgabe wird durch die Bereitstellung eines Arzneimittels gelöst, das zur alkoholischen Gärung befähigte, medizinisch annehmbare Mikroorganismen, wie beispielsweise Hefe, enthält. Die Abgabe des in dem Arzneimittel enthaltene Arzneistoffs wird nach Verabreichung des Arzneimittels durch den verlauf des bei der einsetzenden alkoholischen Gärung entstehenden Kohlendioxids kontrolliert.

Arzneimittel, die Hefezellen enthalten sind bekannt. Beispielsweise sind unter den Handelsnamen Perenterol® und Yomagi® Kapseln erhältlich, die die medizinische Hefe *Saccharamyces boulardii* enthalten. Bei diesen gegen Durchfallerkrankungen einzusetzenden Arzneimittel ist die Hefe selbst als therapeutisch wirksamer Arzneimittelbestandteil anzusehen, da sie dazu dienen soll, die Darmflora zu regenerieren und auf diese Weise dem Durchfall entgegenzuwirken.

Im Stand der Technik sind auch Arzneimittel bekannt, in denen nach ihrer Verabreichung Kohlendioxid gebildet wird. So werden beispielsweise in den Offenlegungsschriften WO 03/0112 A1 und US 2006/003003 A1 gastroretentive Systeme beschrieben, die den durch die Kohlendioxidbildung entstehenden Anftrieb des Arzneimittels nutzen, um auf dem Nahrungsbrei oder der im Magen enthaltenen Flüssigkeit zu schwimmen (flotieren). Dadurch kann eine längere Verweildauer des Arzneimittels im Magen erreicht werden.

Gemäß WO 2006/024638 A2 kann das nach Verabreichung eines Arzneimittels entstehende Kohlendioxid auch genutzt werden, um den Arzneistoff mit der Nahrung im Magen zu mischen, wodurch ein retardierender Effekt ausgelöst werden soll.

Die WO 02/102415 A1 betrifft eine pharmazeutische Zubereitung, die eine gasbildende Komponente zur kontrollierten Freisetzung von Wirkstoff enthält. Die Zubereitung umfasst ein gelbildendes Pulver aus den Hülsen von *Lepidium sativum* Samen. Die Bildung von Gas führt zu Gasblasen in dem sich nach Einnahme der Zubereitung bildenden Gel, das dadurch schwimmfähig wird, so dass der in der Zubereitung enthaltene Wirkstoff über eine längere Zeitdauer im Magen freigesetzt werden kann.

Mit der WO 02/100391 A wird eine Paracetamol enthaltende Tablette oder Kapsel offenbart, die geringe Mengen an Natriumcarbonat oder Kaliumcarbonat als spezifisches Antiacidum zur erhöhten Freisetzung des Wirkstoffs Paracetamol enthält.

Die Erzeugung von Kohlendioxid in Arzneimittel kann auch dazu genutzt werden, den Zerfall der Arzneiform nach ihrer Verabreichung zu beschleunigen, wie beispielsweise in JP 2003 231629 A beschrieben wird.

Die bekannten Arzneimittel, mit denen das nach ihrer Verabreichung entstehende Kohlendioxid genutzt wird, lassen das Kohlendioxid durch Einwirken einer Säure aus einem Salz, üblicherweise Natriumcarbonat, entstehen. Diese chemische Reaktion lässt sich wenn überhaupt nur sehr schwierig kontrollieren.

Die vorliegende Erfindung basiert auf den Überlegungen, daß eine von den äußeren Bedingungen weitgehend unabhängige Arzneistofffreisetzung eine kontrollierbare Kohlendioxiderzeugung erfordern würde, und daß möglicherweise die bei der alkoholischen Gärung erfolgende Kohlendioxidbildung in einem Arzneimittel kontrollierbar sein könnte.

Überraschenderweise haben Untersuchungen der Anmelderin gezeigt, daß sich die Kinetik der Abgabe eines Arzneistoffs aus einem Arzneimittel mittels bei Gärung von Hefe entstehenden Kohlendioxids steuern läßt.
FIG. 1 zeigt den zeitlichen Verlauf der Volumenausdehnung in 20 ml Omnifix®-Spritzen durch Kohlendioxidentwicklung bei der Gärung von Hefe.
FIG. 2 zeigt den zeitlichen Verlauf der Freisetzung von Paracetamol aus einer Modellarzneiform durch bei Gärung von Hefe entstandenes Kohlendioxid.
FIG. 3 zeigt eine schematische Darstellung eines monolithischen, umhüllten Arzneistoff-Freigabesystems basierend auf Zuckersirup, Hefe und Arzneistoff.

Gegenstand der vorliegenden Erfindung sind somit Arzneimittel, die zusätzlich zu dem in ihnen enthaltenen Arzneistoff zur alkoholischen Gärung befähigte, pharmazeutisch annehmbare Hefezellen enthalten. Im Sinne der Erfindung handelt es aich durchweg um eine Vielzahl von Hefezellen, die in den Arzneimitteln enthalten sind, auch wenn im Folgenden der Begriff "Hefe" als Singular verwendet wird.

Die Hefen oder Sproßpilze zählen zu den Protoascomyceten. Die für Hefen im weiteren Sinne typische Art der asexuellen Vermehrung ist die Zellsprossung. Einige Hefen vermehren sich aber auch durch Teilung oder Übergangsformen zwischen Teilung und Sprossung. So können Sproßzellen zu Sproßverbänden oder als Pseudomycel miteinander verbunden bleiben oder sich völlig voneinander lösen, wobei zahlreiche Hefearten auch unseptierte oder septierte Hyphen bilden.

Für die erfindungsgemäßen Arzneimittel sind insbesondere die Hefen geeignet, die in der Back-, Brau- und Weinindustrie verwendet werden. Als Beispiele für diese Hefen können die Saccharomyceten *Saccharomyces cerevisiae*, *Saccharomyces carlsbergensis, Saccharomyces uvarum*, *Saccharomyces boulardii*, *Saccharomyces exiguus und Saccharomyces ladwigii* genannt werden. Es können aber auch Nicht-Saccharomyceten *wie Candida famala, Candida stellala, Dakkera bruxellensis, Hanensula* uvarum, *Kluyveromyces lactis, Kluyveromyces thermotolerans, Metschnikowia pulcherrima oder Torulaspora delbrueckii* verwendet werden.

Vorzugsweise werden Reinhefestämme in dem erfindungsgemäßen Arzneimittel verwendet. In einer bevorzugten Ausführungsform enthält das Arzneimittel die Bäckerhefe (*Saccharomyces cerevisiae*). Es ist aber auch möglich, zwei oder mehrere Hefestämme in Kombination miteinander zu verwenden, um den Verlauf der Gärung im Arzneimittel zu steuern.

Grundsätzlich ist es möglich, daß das Arzneimittel die Hefe als Frischhefe enthält. Besonders bevorzugt wird die Hefe jedoch in Form von Instant-Trockenhefe für die Herstellung der Arzneimittel verwendet.

Bei der alkoholischen Gärung wird von der Hefe unter anaeroben Bedingungen Glucose zu Ethanol und Kohlendioxid verstoffwechselt:

C₆H₁₂O₆ → 2 C₂H₅OH + 2 CO₂

Glucose ist somit ein essentielles Edukt für die Alkoholische Gärung. Bei besonderen Ausführungsformen des erfindungsgemäßen Arzneimittels kann davon Gebrauch gemacht werden, daß der Nahrungsbrei im Verdauungstrakt Kohlenhydrate und insbesondere Glucosen enthält, die von den Hefen in dem Arzneimittel zur Gärung genutzt werden können. Bei diesen Ausführungsformen ist es nicht notwendig, daß das erfindungsgemäße Arzneimittel auch den für die Gärung erforderlichen Zucker enthält, sofern der im Nahrungsbrei vorhandene Zucker in das Arzneimittel gelangen kann.

Bei bevorzugten Ausführungsformen enthält das erfindungsgemäße Arzneimittel jedoch auch den/die für die Gärung erforderlichen Kohlenhydrate. Besonders bevorzugt enthält das Arzneimittel Glucose.

Anstelle von Glucose oder zusätzlich zu Glucose können auch andere Zucker oder deren Mischungen verwendet werden, die die Hefen im Arzneimittel für ihre Gärung nutzen können. Beispielsweise können auch Fructose, Galactose, Saccharose, Maltose, Maltotriose, Raffinose oder beliebige Mischungen dieser Zucker in dem Arzneimittel verwendet werden. Zudem können auch Stärke oder Stärkederivate, beispielsweise Dextrine, verwendet werden.

Die Arzneimittel weisen einen Überzug auf. Als Materialien für die Herstellung der Überzüge kommen in Betracht:
- Celluloseether, beispielsweise
   Hydroxypropylmethylcellulose oder Ethylcellulose,
- Celluloseester, beispielsweise Celluloseacetat, Celluloseacetatbutyrat oder Celluloseacetatpropionat,
- Polyacrylate und Polymethacrylate, beispielsweise die im Handel unter den Warenzeichen Eudragit® RS, Eudragit® RL oder Eudragit® NE erhältlichen Produkte,
- Polyvinylderivate, wie zum Beispiel Polyvinylacetat,
- Copolymerisate aus Polymethylvinylether und Maleinsäure oder deren Ethyl-, Isopropyl- und n-Butylester, beispielsweise das im Handel unter dem Warenzeichen Gantrez® AN erhältliche Produkt.

Es können auch Mischungen der vorgenannten Polymere für die Überzüge verwendet werden, beispielsweise Ethylcellulose und Hydroxypropylcellulose in einem Gewichtsverhältnis von 60:40.

Darüber hinaus können dem Überzug geeignete Hilfsstoffe beigefügt sein, mit denen die Eigenschaften der Überzüge beeinflußt werden können. Als Hilfsstoffe kommen beispielsweise Weichmacher, Netzmittel oder Pigmente in Frage. Als Weichmacher können beispielsweise Ester wie Triethylcitrat, Tributylcitrat, Acetyltriethylcitrat, Dibutyltartrat, Diethylsebacat, Dimethylphthalat, Diethylphthalat, Dioctylphthalat, Rizinusöl, Sesamöl, Glycerintriacetat, Glyceringdiacetat, höhere Alkohole, beispielsweise Glycerin oder 1,2-Propylenglykol, oder Polyether, beispielsweise Polyethylenglykole verwendet werden.

Geeignete Netzmittel sind zum Beispiel PEG-400-Stearat, Sorbitanmonooleat und PEG-Sorbitanmonooleat.

Geeignete Pigmente sind beispielsweise Titandioxid und Eisenoxide.

Durch den Zusatz derartiger Hilfsstoffe können die Eigenschaften der Überzüge beeinflußt werden, da die mechanischen Eigenschaften wie Flexibilität, Sprödigkeit und Festigkeit, sowie die Schichtdicke des Überzugs die Arzneistofffreisetzung beeinflussen.

So kann das erfindungsgemäße Arzneimittel in einer Ausführungsform analog einem osmotisch gesteuerten Abgabesystem aufgebaut sein, so daß das durch die Gärung entstehende Kohlendioxid eine Arzneistofflösung oder Arzneistoffsuspension durch eine Abgabeöffnung in dem Arzneimittel nach außen drückt. Auf diese Weise kann eine kontinuierliche Arzneistoffabgabe, die sich über einen längeren Zeitraum erstreckt, realisiert werden.

Für diese Ausführungsform wird ein Überzug aus Celluloseacetat bevorzugt, da er sich durch eine besondere Festigkeit auszeichnet.

In anderen Ausführungsformen kann das Arzneimittel ein Abgabesystem sein, welches den Arzneistoff schlagartig oder pulsatil freisetzt. Dabei kann die Freisetzung des Arzneistoffs vergleichsweise schnell oder auch erst nach einer zeitlichen Verzögerung erfolgen. Um die Freisetzung des Arzneistoffs zu bestimmen, kann das System monolithisch oder aus mehreren Einzeleinheiten (multiple units) bestehen. Sofern alle Einzeleinheiten die gleichen Freinetzungseigenschaften aufweisen, kann eine schlagartige Freisetzung des in den Einzeleinheiten enthaltenen Arzneistoffs erfolgen (burst release). Weisen die Einzeleinheiten unterschiedliche Freisetzungseigenschaften auf, kann eine pulsatile Freisetzung des Arzneistoffs möglich werden.

Abhängig von den Eigenschaften des Überzugs kann die Hefe unmittelbar nach Verabreichen der Arzneiform mit der Gärung beginnen oder erst mit Verzögerung, so daß auch erfindungsgemäße Arzneimittel mit verzögerter Arzneistofffreisetzung möglich sind.

Für Abgabesysteme mit schlagartiger, pulsatiler und/oder verzögerter Arzneistofffreisetzung werden Überzüge aus Ethylcellulose oder auf Basis von Ethylcellulose bevorzugt, deren Eigenschaften mit Hilfe von Weichmachern und/oder einer Änderung der Schichtdicke modifiziert werden können. Beispielsweise erhöht die Zugabe eines Weichmachers wie Triethylcitrat die Flexibilität und Festigkeit des Überzugs stärker als der Zusatz von Dibutylsebacat.

Insbesondere bei der Verwendung von Trockenhefe ist Wasser zur Rehydratation der Hefe erforderlich. Das Wasser kann der Arzneiform von außen zugeführt werde, entweder bei der Einnahme der Arzneiform oder als bereits im Gastrointestinaltrakt befindlich. Bei einer besonderen Ausführungsform ist das für die Rehydratation benötigte Wasser bereits in der Arzneiform enthalten. Um eine vorzeitige Aktivierung der Hefe und ihrer Gärung zu vermeiden, ist das Wasser zunächst von der Hefe getrennt zu halten, in dem es sich in einem separaten Kompartiment bebindet. Um die Hefe zu aktivieren, muß die Wand des wasserhaltigen Kompartiments beispielsweise durch Zerdrücken, zerstört werden. Dadurch gelangt das Wasser in Kontakt mit der Hefe und kann diese rehydrieren und die Gärung in Gang setzen.

### Beispiel 1

Um zu untersuchen, ob bereits geringe Mengen an Edukten ausreichen, genügend Kohlendioxid zu produzieren, um eine Arzneistofffreisetzung realisieren zu können, wurden zwischen 25 mg und 100 mg Hefe, bis zu 100 mg GlucoseMonohydrat und 500 µl gereinigtes Wasser miteinander vermischt und in 20 ml Einwegspritzen (Omnifix®) gefüllt, deren Injektionsöffnung verschlossen war. Nach Einsetzen des Stempels wurden die befüllten Spritzen bei 37 °C inkubiert. Als Maß für die Kohlendioxidproduktion wurde die Stempelbewegung protokolliert, welche die Volumenzunahme in der Spritze anzeigt.

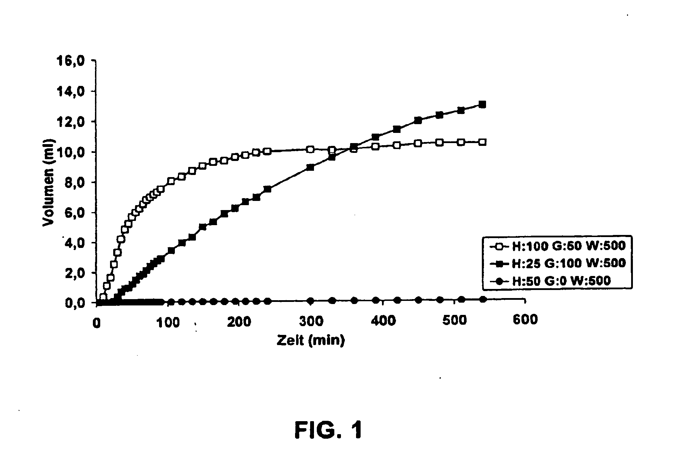

Die Ergebnisse dieses Versuchs sind in FIG. 1 dargestellt, in der die eingesetzten Mengen an Hefe (H) in mg, an Glucose-Monohydrat (G) in mg und an Aqua purificata (W) in µl angegeben sind. Die angegebenen Werte sind die Mittelwerte aus 6 Einzelwerten. Neben dem zeitlichen Verlauf der Volumenausdehnung ist ersichtlich, daß das Verhältnis der eingesetzten Mengen an Hefe und Glucose einen EinfluB auf die Kinetik der Kohlendioxidproduktion hat. Dieses kann bei der Herstellung von Arzneimitteln genutzt werden, um die Freisetzungsrate des in dem Arzneimittel enthaltenen Arzneistoffs einzustellen.

### Beispiel 2

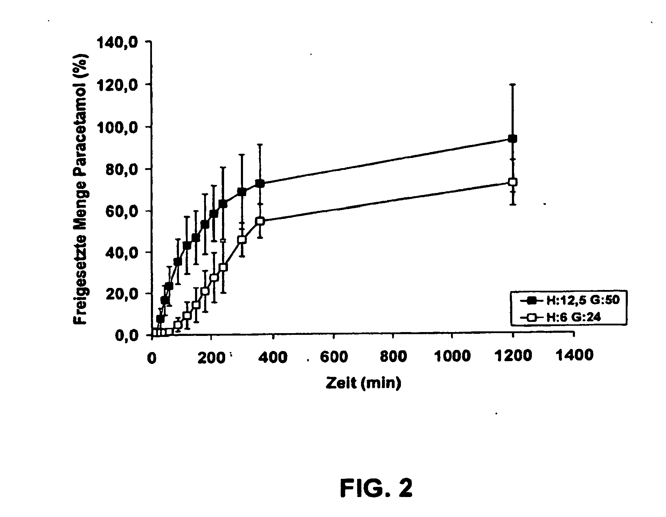

Mit einem weiterführenden Versuch sollte festgestellt werden, ob ein Arzneistoff mit Hilfe des bei der Gärung entstehenden Kohlendioxids über einen ausreichend langen Zeitraum freigesetzt werden kann. Dazu wurde Paracetamol in einer Mischung aus Polyethylenglykol und hochdispersem Siliciumdioxid in eine Einwegspritze gefüllt. Die Mischung wurde mit einem in die Spritze eingesetzten Plättchen abgedeckt, anschließend wurde eine Mischung aus Hefe, Glucose und Wasser auf das Plättchen gegeben und die Spritze wurde an der Einfüllöffnung verschlossen. Die jeweils eingesetzten Mengen an Hefe (H) in mg, an Glucose-Monohydrat (G) in mg sind der Legend zu FIG. 2 zu entnehmen. Die zur Aktivierung verwendete Wassermenge betrug 125 µl.

Die so gebildete Modellarzneiform wurde in einem Dissolutionstester bei 100 UpM und 37°C in 900 ml Wasser inkubiert, und die in das umgebende Wasser abgegebene Menge Paracetamol wurde zu verschiedenen Zeitpunkten bestimmt.

Die Ergebnisse dieser Versuche sind in FIG. 2 dargestellt. Die dargestellten Meßwerte stellen den Mittelwert aus n=3 dar.

Es zeigte sich, daß der Arzneistoff mit Hilfe von durch Gärung erzeugtem Kohlendioxid über einen längeren Zeitraum hinweg aus der Modellarzneiform freigesetzt werden kann. Zudem ist die Kinetik der Arzneistofffreisetzung abhängig von der verwendeten Menge Hefe. Somit kann die Freisetzungsrate für den Wirkstoff durch die Menge an Hefe und/oder Glucose in der Arzneiform gesteuert werden.

### Beispiel 3

### "Osmotisch" gesteuertes Abgabesystem

Analog einem osmotisch gesteuerten Abgabesystem kann durch entstehendes Kohlendioxid ein Druck in der Arzneiform aufgebaut werden, der eine Arzneistofflösung/-suspension durch eine Abgabeöffnung nach außen drückt. Ein derartiges Abgabesystem wurde wie folgt hergestellt:

### Wirkstoffschicht

| | | |
|---|---|---|
| Innere Phase | Wirkstoff | 22,5 Gew.-% |
| | Hydroxypropylmethylcellulose | 6,0 Gew.-% |
| | Polyethylenoxid | 70,0 Gew.-% |
| Äußere Phase | Magnesiumstearat | 1,0 Gew.-% |
| | hochdisperses Siliciumdioxid | 0,5 Gew.-% |
| | | |
| Expandierende | Schicht | |
| Innere Phase | Polyethylenoxid | 49,0 Gew.-% |
| | Glucose-Monohydrat | 40,0 Ges.-% |
| | Trockenhefe | 10,0 Gew.-% |
| Äußere Phase | Magnesiumstearat | 1,0 Gew.-% |

Die Bestandteile der jeweils inneren Phase beider Schichten wurden separat granuliert. Anschließend wurde die jeweilige äußere Phase zugerüstet und beide Granulate wurden zu einer bikonvexen Zweischichttablette verpreßt. Die erhaltenen Tabletten wurde mit einem für Wasser permeablen, gasdichten Überzug versehen, für den 20 g Celluloseacetat in 970 ml Aceton gelöst und mit 4,5 g in 30 ml Wasser gelöstem Polyethylenglykol 400 vermischt worden waren. Anschließend wurde eine Öffnung für die Arzneistoffabgabe im Bereich der wirkstoffhaltigen Schicht der Zweischichttablette in den Überzug gebohrt.

### Beispiel 4

Ein Abgabesystem mit schlagartiger Wirkstofffreisetzung wurde wie folgt hergestellt:

| Zuckersirup | |
|---|---|
| Glucose-Monohydrat | 71,4 % |
| Gereinigtes Wasser | 28,6 % |
| | |

| Arzneiform | |
|---|---|
| Zuckersirup | 40 % |
| Mikronisierte Trockenhefe | 40 % |
| Arzneistoff Paracetamol | 20 % |

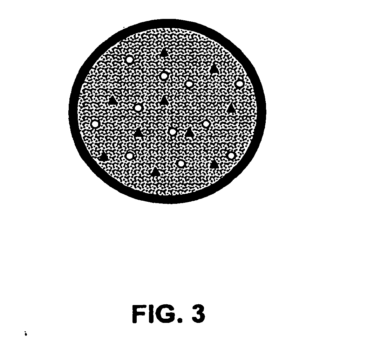

Zunächst wurde der Zuckersirup hergestellt. In diesen wurde beim Erkalten die Trockenhefe und der Arzneistoff eingearbeitet, und es wurde kleine Kugeln geformt, die mit einem wasserpermeablen, möglichst gasdichten Überzug versehen wurden, für den 20 g Ethylcellulose mit 4 g Dibutylsebacat in 200 ml Ethanol gelöst worden waren. Dieses Arzneistoff-Freigabesystem mit Hefe (▲) und Arzneistoff (○) wird in FIG. 3 schematisch dargestellt.

## Patentansprüche

1. Arzneimittel zur kontrollierten Freisetzung des im Arzneimittel enthaltenen Arzneistoffs, **dadurch gekennzeichnet, daß** das Arzneimittel eine medizinisch unbedenkliche, zur alkoholischen Gärung befähigte Hefe und einen Arzneistoff enthält.

2. Arzneimittel nach Anspruch 1, **dadurch gekennzeichnet, daß** die Hefe aus der Gruppe von Hefen ausgewählt ist, die aus *Saccharomyces cerevisiae, Saccharomyces carlsbergensis*, *saccharomyces uvarum, saccharomyces boulardii, Saccharomyces exiguus und Saccharomyces ludwigii, Candida famala, Candida stellala, Dekkera bruxellensis, Hanensula uvarum, Kluyveromyces lactis, Kluyveromyces thermotolerans, Metschnikowia pulcherrima, Torulaspora delbrueckii* und deren Mischungen besteht.

3. Arzneimittel nach Anspruch 1 oder 2, **dadurch gekennzeichnet, daß** es sich bei der Hefe um Frischhefe oder Trockenhefe handelt.

4. Arzneimittel nach einem der vorangehenden Ansprüche, **dadurch gekennzeichnet, daß** das Arzneimittel ferner einen oder mehrere Kohlenhydrate enthält, die vorzugsweise aus der Gruppe ausgewählt ist/sind, die aus Glucose, Fructose, Galactose, Saccharose, Maltose, Maltotriose, Raffinose, Stärke, Starkederivaten und Dextrinen besteht.

5. Arzneimittel nach einem der vorangehenden Ansprüche, **dadurch gekennzeichnet, daß** das Arzneimittel zusätzlich Wasser in einem separaten Kompartiment enthält.

6. Arzneimittel nach einem der vorangehenden Ansprüche, **dadurch gekennzeichnet, daß** *das* Arzneimittel einen Überzug aufweist, der vorzugsweise ein Material umfaßt, das aus der Gruppe ausgewählt ist, die aus Celluloseether, celluloseester, Polyacrylat, Polymethacrylat, Polyvinylderivat und Copolymerisat aus Polymethylvinylether und Maleinsäure besteht.

7. Arzneimittal nach Anspruch 6, **dadurch gekennzeichnet, daß** das Material für den Überzug einen oder mehrere Hilfsstoffe aufweist, die vorzugsweise aus der Gruppe der Weichmacher, Netzmittel und Pigmente ausgewählt ist.

8. Arzneimittel nach Anspruch 7, **dadurch gekennzeichnet, daß** der Weichmacher aus der Gruppe ausgewählt ist, die aus Triethylcitrat, Tributylcitrat, Acetyltriethylcitrat, Dibutyltartrat, Biethylsebacat, Dimethylphthalat, Diethylphthalat, Dioctylphthalat, Rizinusöl, Sesamöl, Glycerintriacetat, Glycerindiacetat, Glycerin, 1,2-Propylenglykol und Polyethylenglykolen besteht.

9. Arzneimittel nach Anspruch 7 oder 8, **dadurch gekennzeichnet, daß** das Netzmittel aus der Gruppe ausgewählt ist, die aus PEG-400-Stearat, Sorbitanmonooleat und PEG-sorbitanmonooleat besteht.

10. Arzneimittel nach einem der Ansprüche 7 bis 9, **dadurch gekennzeichnet, daß** das Pigment aus der Gruppe ausgewählt ist, die Titandioxid und Eisenoxide umfaßt.

11. Verfahren zur Herstellung eines Arzneimittels für eine kontinuierliche Arzneistofffreigabe, **dadurch gekennzeichnet, daß** eine wirkstoffhaltige Schicht und eine Hefe enthaltende Schicht zu eine Zweischichttablette verpreßt werden, die zweischichttablette mit einem für Wasser permeablen, gasdichten Überzug versehen wird, und eine Öffnung in dem Überzung im Bereich der wirkstoffhaltigen Schicht gebohrt wird.

12. Verfahren zur Herstellung eines Arzneimittels für eine schlagartige Arzneistofffreisetzung, **dadurch gekennzeichnet, daß** ein Arzneistoff und Hefe in einen Zuckersirup eingearbeitet werden, und die so erhaltene Mischung mit einem wasserpermeablen, möglichst gasdichten Überzug versehen wird, nachdem kleine Kugeln geformt wurden

13. Verwendung einer medizinisch unbedenklichen, zur alkoholischen Gärung befähigten Hefe für die Herstellung eines Arzneimittel zur kontrollierten Freisetzung des im Arzneimittel enthaltenen Arzneistoffs.

## Claims

1. Pharmaceutical product for controlled release of the active pharmaceutical ingredient it contains, **characterised in that** said pharmaceutical product comprises a medically acceptable yeast that is capable of alcoholic fermentation, and an active pharmaceutical ingredient.

2. Pharmaceutical product according to claim 1, **characterised in that** said yeast is selected from the group of yeasts which consists of *Saccharomyces cerevisiae, Saccharomyces carlsbergensis, Saccharomyces uvarum, Saccharomyces boulardii, Saccharomyces exiguus* and *Saccharomyces ludwigii, Candida famala,* Candida *stellala, Dekkera bruxellensis, Hanensula uvarum, Kluyveromyces lactis, Kluyveromyces thermotolerans, Metschnikowia pulcherrima, Torulaspora delbrueckii* and mixtures thereof.

3. Pharmaceutical product according to claim 1 or 2, **characterised in that** said yeast is fresh yeast or dry yeast.

4. Pharmaceutical product according to any one of the preceding claims, **characterised in that** said pharmaceutical product moreover contains one or more carbohydrates which is/are preferably selected from the group consisting of glucose, fructose, galactose, saccharose, maltose, maltotriose, raffinose, starch, starch derivatives and dextrins.

5. Pharmaceutical product according to any one of the preceding claims, **characterised in that** said pharmaceutical product in addition contains water within a separate compartment.

6. Pharmaceutical product according to any one of the preceding claims, **characterised in that** said pharmaceutical product has a coating which preferably comprises a material selected from the group consisting of cellulose ethers, cellulose esters, polyacrylate, polymethacrylate, polyvinyl derivative, and copolymer of polymethyl vinyl ether and maleic acid.

7. Pharmaceutical product according to claim 6, **characterised in that** the material for said coating comprises one or more excipients which is/are preferably selected from the group of the plasticisers, wetting agents and pigments.

8. Pharmaceutical product according to claim 7, **characterised in that** the plastiziser is selected from the group consisting of triethyl citrate, tributyl citrate, acetyl triethyl citrate, dibutyl tartrate, diethyl sebacate, dimethyl phthalate, diethyl phthalate, dioctyl phthalate, castor oil, sesame seed oil, glyceryl triacetate, glyceryl diacetate, glycerol, 1,2-propylene glycol and polyethylene glycols.

9. Pharmaceutical product according to claim 7 or 8, **characterised in that** the wetting agent is selected from the group consisting of PEG 400 stearate, sorbitan monooleate and PEG sorbitan monooleate.

10. Pharmaceutical product according to any one of claims 7 to 9, **characterised in that** the pigment is selected from the group comprising titanium oxide and iron oxides.

11. Method for manufacturing a pharmaceutical product for a continuous release of active pharmaceutical ingredient, **characterised in that** an active ingredient-containing layer and a yeast-containing layer are compressed into a bilayer tablet, that the bilayer tablet is provided with a water-permeable gas-tight coating, and that an opening is drilled in the coating in the region of the active ingredient-containing layer.

12. Method for manufacturing a pharmaceutical product for a burst release of active pharmaceutical ingredient, **characterised in that** an active pharmaceutical ingredient and yeast are incorporated in a sugar syrup, and that the mixture thus obtained is provided with a water-permeable coating that is as gas-tight as possible, after the forming of small spheres.

13. Use of a medically acceptable yeast that is capable of alcoholic fermentation, for the manufacturing of a pharmaceutical product for the controlled release of the active pharmaceutical ingredient contained in said pharmaceutical product.

## Revendications

1. Médicament permettant une libération contrôlée du principe actif contenu dans ledit médicament, **caractérisé en ce que** ledit médicament contient une levure médicalement acceptable qui est apte à la fermentation alcoolique, et un principe actif.

2. Médicament selon la revendication 1, **caractérisé en ce que** ladite levure est choisie dans le groupe des levures constitué de *Saccharomyces cerevisiae, Saccharomyces carlsbergensis, Saccharomyces uvarum, Saccharomyces boulardii, Saccharomyces exiguus* et *Saccharomyces ludwigii, Candida famala, Candida stellala, Dekkera bruxellenses, Hanensula uvarum, Kluyveromyces lactis, Kluyveromyces thermotolerans, Metschnikovia pulcherrima, Torulaspora delbrueckii* et de leurs mélanges.

3. Médicament selon les revendications 1 ou 2, **caractérisé en ce que** ladite levure est une levure fraîche ou une levure sèche.

4. Médicament selon l'une des revendications précédentes, **caractérisé en ce que** ledit médicament contient en outre un ou plusieurs glucides, choisi(s) de préférence dans le groupe constitué de glucose, de fructose, de galactose, de saccharose, de maltose, de maltotriose, de raffinose, d'amidon, de dérivés d'amidon et de dextrines.

5. Médicament selon l'une des revendications précédentes, **caractérisé en ce que** ledit médicament contient en outre de l'eau dans un compartiment séparé.

6. Médicament selon l'une des revendications précédentes, **caractérisé en ce que** ledit médicament présente un enrobage comprenant de préférence un matériau choisi dans le groupe constitué d'éther de cellulose, d'ester de cellulose, de polyacrylate, de polyméthacrylate, d'un dérivé polyvinylique et d'un produit issu de copolymérisation de polyméthylvinyléther et d'acide maléique.

7. Médicament selon la revendication 6, **caractérisé en ce que** ledit matériau destiné à l'enrobage comporte un ou plusieurs agents auxiliaires choisis de préférence dans le groupe des plastifiants, des agents mouillants et des pigments.

8. Médicament selon la revendication 7, **caractérisé en ce que** ledit plastifiant est choisi dans le groupe constitué de citrate de triéthyle, de citrate de tributyle, d'acétylcitrate de triéthyle, de tartrate de dibutyle, de sébaçate de diéthyle, de phtalate de diméthyle, de phtalate de diéthyle, de phtalate de dioctyle, d'huile de ricin, d'huile de sésame, de triacétate de glycérol, de diacétate de glycérol, de glycérol, de 1,2-propylène glycol et de polyéthylèneglycols.

9. Médicament selon les revendications 7 ou 8, **caractérisé en ce que** ledit agent mouillant est choisi dans le groupe constitué de stéarate de PEG-400, de monooléate de sorbitane et de monooléate de PEG-sorbitane.

10. Médicament selon l'une des revendications 7 à 9, **caractérisé en ce que** ledit pigment est choisi dans le groupe comprenant le dioxyde de titane et des oxydes de fer.

11. Procédé de fabrication d'un médicament destiné à une libération contrôlée du principe actif, **caractérisé en ce qu'**une couche contenant des principes actifs et une couche contenant de la levure sont soumises à une pression pour former un comprimé bicouche, ledit comprimé bicouche est pourvu d'un enrobage étanche aux gaz et perméable à l'eau, et un orifice est percé dans ledit enrobage au niveau de la couche contenant des principes actifs.

12. Procédé de fabrication d'un médicament destiné à une libération instantanée du principe actif, caractérisé en ce qu'un principe actif et de la levure sont incorporés dans un sirop de sucre, et le mélange ainsi obtenu est d'abord transformé en petites billes puis pourvu d'un enrobage qui est permeable à l'eau et, dans la mesure du possible, étanche aux gaz.

13. Utilisation d'une levure médicalement acceptable et apte à la fermentation alcoolique, pour fabriquer un médicament destiné à la libération contrôlée du principe actif contenu dans ledit médicament.
